# EUROPEAN PATENT SPECIFICATION

(11) **EP 4 181 978 B1**
(45) Date of publication and mention of the grant of the patent: **01.01.2025**
(21) Application number: 22712622.4
(22) Date of filing: 08.02.2022
(51) Int. Cl.: A61L 15/58, G01N 33/52, G01N 33/84

(54) **HOT MELT ADHESIVE TO DETECT ANALYTES IN URINE**
SCHMELZKLEBSTOFF ZUM NACHWEIS VON ANALYTEN IN URIN
ADHÉSIF THERMOFUSIBLE POUR DÉTECTER DES ANALYTES DANS L'URINE

(30) Priority: 28.01.2022 PT 2022117766; 03.02.2022 PT 2022117775
(43) Date of publication of application: 24.05.2023
(73) Proprietor: Colquimica-Indústria Nacional de Colas, S.A., 4440-578 Valongo (PT)
(72) Inventor: FRUTUOSO, Cristina, 4450-150 Matosinhos (PT); SOARES, Pedro, 4100-271 Porto (PT); PIMENTA, Ana, 4580-575 Lordelo Prd (PT)
(74) Representative: Moniz Pereira, Manuel
(86) International application number: PCT/IB2022/051108
(87) International publication number: WO 2023/057821

(56) References cited:
- WO-A1-97/48779
- CN-U- 214 967 755
- US-A1- 2008 269 707
- US-A1- 2009 155 122

## Description

### Field of the invention

The present invention relates to a hot melt adhesive composition, more specifically, to a hot melt that detects analytes in urine, and the hot melt is applied in personal hygiene disposable articles.

### Background of the invention

Urine is an aqueous solution secreted by the kidneys composed by more than 950 of water. The remaining components of healthy urine are urea, chloride, sodium, potassium, creatinine, and dissolved ions.

Abnormal components or abnormal characteristics of urine are usually indicative of health issues, such as urinary tract infections. These infections occur when bacteria reach the urinary tract through the urethra and start to multiply in the bladder. Some individuals are more prone to develop a urinary tract infection than others. For example, women are more affected by urinary tract infections due to the shorter urethra compared to men. Diseases that suppress the immune system, such as diabetes and urinary tract abnormalities, common in babies, increase the risk to develop an infection. Studies reveal that around 20% of women with age over 65 years have a urinary tract infection, against 11% in the overall population and the prevalence of the infection increases with age.

Although common, most urinary tract infections are uncomplicated, without relevant functional or anatomical abnormalities. Nevertheless, medical treatment is always recommended due to the risk of recurrent infections or to develop a more serious infection that can potentially damage the kidneys. The dominant infectious agent of both uncomplicated and complicated urinary tract infections is *Escherichia coli* (E. coli), a gram-negative bacterium.

There are several physiological parameters that change in the presence of a urinary tract infection, such as the change in colour, clarity, odour, and/or pH of urine, as well as the possible presence of proteins, nitrites, ketones, glucose, and/or blood cells in urine. From these components, the presence of nitrites can be used to detect a urinary tract infection as the bacteria that causes the infection produces nitrate reductases, enzymes that convert nitrates into nitrites.

While also present in urinary tract infections, other analytes such as glucose and proteins are also indicative of other pathologies, such as type 2 diabetes and kidney diseases, respectively. Diabetes is characterized by the presence of excess glucose in blood, which is removed through the urine, as a method to remove such excess from the body. Kidney diseases can be associated with the destruction of nephrons, such as in nephrotic syndrome, which causes blood proteins and other proteins associated with body damage, e.g. C-reactive protein, to pass to the urine in higher amounts. Other analytes can also pass to the urine in unhealthy amounts, such as sodium, chloride, potassium, creatine, creatinine, calcium, phosphates and others, with the passage of creatinine being smaller in the presence of kidney complications.

For some of these analytes, methods of rapid detection already exist. For nitrite, the detection is done by a rapid screening method for possible asymptomatic infections caused by nitrate-reducing bacteria. For glucose, the main method used to check for diabetes is a blood-glucose meter, which is characterised by a sting in the finger, drawing blood to the detector and measuring the amount of glucose present in the blood.

For other analytes, quantitative analysis is still the most ideal method of detecting their presence. For creatine and creatinine, chromatography methods are mainly used. For proteins, the total protein test is applied.

Rapid screen tests, while faster, cannot replace the urinalysis tests nor microscopic examination as diagnostic tools, nor subsequent monitoring. The presence of these analytes can be associated with other complications not associated with kidney function, therefore requiring methods of quantification. For the case of urinary tract infection, many other microorganisms that do not reduce nitrate (such as gram-positive bacteria and yeasts) can also cause urinary infections.

Nevertheless, the usage of technologies that enable the detection of analytes in urine presents various advantages to the patients. The most notable benefit is the quick preliminary diagnosis. By observing an indication of a possible health problem, patients can request the attention of a general practitioner, being then further directed to a doctor that specializes in the type of condition that the patient might present. In this way, more efficient diagnostic methods directed to confirming preliminary diagnosis can be applied, effectively shortening the amount of tests done and time spent on diagnosing the condition. Such allows for a faster start to treatments, thereby preventing further health issues derived from the initial pathology.

The present invention relates to a hot melt adhesive composition able to detect urinary tract complications, by detecting the presence of analytes in urine, and where the hot melt is applied in personal hygiene disposable articles. The detection of analytes is possible due to the incorporation of a reagent. This reagent reacts with a specific analyte in samples to form a coloured compound.

By incorporating such reagent in the hot melt adhesive composition, the product of the invention will change its colour in the presence of the analyte. Therefore, a possible urinary tract complication is detected, allowing for earlier treatments to begin. Among the patients who need a quick diagnosis of urinary tract complications, newborns and babies are the most critical due to the inability to communicate and the difficulty of collecting the urine. Additionally, bedridden individuals also tend to present difficulties communicating the presence of symptoms to their caregivers, which delays treatments and can significantly worsen the consequences to their already compromised health. In these cases, the application of the hot melt adhesive of the present invention in the backsheet area of hygiene disposable articles, namely, but not exclusively, diapers and sanitary napkins, would allow for an easier identification of individuals with urinary tract complications. Additionally, due to the light sensibility of the adhesive, the present invention also relates to the process of how to protect the hot melt from the light throughout its production and application processes.

Additionally, the use of the hot melt adhesive in other types of disposable articles, such as, but not exclusively, bandages, cotton swabs and dressings, can also be performed.

### Background of the invention

In 1964 the company Boehringer Mannheim, today Roche, launched its first Combur test strips. Even though the test strips have changed little in appearance since the 1960s, they now contain several innovations. New impregnation techniques, more stable colour indicators, and the steady improvement in colour gradation have all contributed to the established use of urine test strips in clinical and general practice as a reliable diagnostic instrument.

Nowadays, there are several commercial kits to detect the presence of analytes, based on a visual change in colour, such as the urine test strip or dipstick. The urine test method consists of immersing the test strip completely in a well-mixed sample of urine for a short period of time, then extracting it from the container and supporting the edge of the strip over the mouth of the container to remove excess urine. The strip is then left to stand for the necessary time for the reactions to occur (usually 1 to 2 minutes), and finally the colours that appear are compared against the chromatic scale provided by the manufacturer.

One of the disadvantages of this method is that an improper technique can produce false results, for example, leukocytes and erythrocytes precipitate at the bottom of the container and may not be detected if the sample is not properly mixed. In the same way, if an excess of urine remains on the strip after it has been removed from the test sample, it may cause the reagents to leak from the pads onto adjacent pads resulting in mixing and distortion of the colours.

Some patent documents were found that refer to techniques for the detection of analytes in urine. For instance, document WO2021195657A1 refers to a sensor that can detect the presence of analytes in bodily fluids, such as nitrites, and can be incorporated in hygiene disposable articles. However, this document regards to an electronic invention, and not to a hot melt adhesive.

CN 214967755U relates to a diaper for detection of human health status by urine testing. The analyte detection layer can be in the form of a hot melt adhesive which is arranged on the PE bottom film and which changes colour.

### Advantages of the invention

It is evident that the state of the art has evolved to keep pace with increasingly demanding technologies. Personal hygiene disposable articles that can detect the presence of analytes would be very useful for hospitals and nursing homes, since it would allow a quick detection of urinary tract complications and, therefore, allow an early diagnosis of the disease, thereby avoiding other health consequences that can derive from these complications.

In accordance with the state of the art, it is intended that the product of this invention is suitable for the most demanding technologies in personal hygiene disposable products. The present invention signals the presence of a urinary tract complication by a colour change of the hot melt adhesive from, for instance, white to red in the presence of nitrites. The stronger the colour intensity, the higher concentration of the analyte.

Furthermore, the biggest advantage observable from the use of the hot melt adhesive composition with the ability to detect the presence of analytes in urine is that, when applied in hygiene disposable products, it allows for fast detection of signs related to urinary tract complications without the patient having to communicate any symptoms to their caregiver. As mentioned before, this would be of extreme importance to newborn babies and young children, as well as bedridden adults, who are unable to communicate. Additionally, the presence of the hot melt adhesive has no disadvantages to the patient, since it is part of the hygiene product, being unnoticeable to senses such as smell or touch.

### Detailed description of the invention

The scope of protection is defined by the claims.

The present invention relates to a hot melt adhesive according to claim 1. It changes its colour in the presence of a solution or bodily fluids, such as, but not exclusively, urine, that contain analytes in its composition. In a preferred embodiment, the hot melt adhesive goes from white to red or pink, and the colour intensity depends on the concentration of the analyte being analysed.

By "analyte" is meant any biochemical compound excreted through the urine that is associated with a pathology, such as nitrite, glucose, proteins and other such compounds.

By "open time" is meant the maximum period of time after the adhesive is applied in the first substrate in which a second substrate can be effectively bonded to the first substrate.

By "substrate" is meant the material or the material surface in which the hot melt adhesive is applied and/or contacts directly with the material. In the context of personal hygiene disposable articles, namely diapers, typical substrates are polyethylene films which are also referred to as "backsheets".

By "setting time" is meant the minimum period of time necessary to compress two or more substrates to achieve a strong bond. Thus, the setting time is related with the recovery speed of the adhesive cohesive strength.

By "affinity for water", also known as hydrophilicity, is meant the ability to establish intermolecular bonds with water molecules. This property, typical of polar molecules, defines the attraction and interaction of different molecules with water molecules.

By "extremely slow colour change" is meant a colour transition that takes more than 10 minutes to occur or be completed.

By "instant colour change" is meant a colour transition that takes less than 1 minute to occur or be completed.

Preferably the hot melt adhesive comprises:
- a polymeric component comprising at least one thermoplastic polymer that is partially soluble in water to allow the contact of urine, and therefore the nitrites, with the reagent component;
- a resin component comprising at least one hydrocarbon resin;
- a plasticiser component comprising at least one plasticiser capable of producing a homogeneous mixture with the polymeric component and with the ability to adjust rheological properties, such as viscosity.
- a surfactant component comprising at least one non-ionic surfactant;
- a reagent component capable of reacting with an analyte resulting in a colour change;
- an antioxidant component comprising at least one phenolic antioxidant.

Additionally, the composition of the present invention can present other components, such as, but not exclusively, oils, stabilizers, pigments, dyestuffs, antiblock additives, polymeric additives, defoamers, preservatives, thickeners, rheology modifiers, humectants, fillers, solvents, nucleating agents, chelating agents, gelling agents, processing aids, cross-linking agents, neutralizing agents, flame retardants, fluorescing agents, compatibilizers, antimicrobial agents, and water.

### Polymeric component

By "polymeric component" is meant the raw materials that contribute to the main properties of the hot melt adhesive, considered the backbone of the composition. Therefore, the remaining components of the invention composition are intended to modify or enhance properties of the polymeric component.

The solubility or affinity for water of the hot melt adhesive is essential in assuring a good performance. By being partially soluble, urine can diffuse through the hot melt adhesive and, therefore, allow contact between the analyte and the reagent component, which will result in a change of colour of the hot melt adhesive. If the hot melt is insoluble or not soluble enough, the colour change will not occur or will be very slow. On the other hand, if the composition of the hot melt adhesive is too soluble, the colour change will be instantaneous and the adhesive may fade, which is not desired. In this context, a colour change that takes up to 10 minutes to occur is considered acceptable. Thus, the composition solubility must be carefully fine-tuned to achieve the desired properties.

Since the invention relates to a hot melt adhesive, properties such as adhesion, cohesion, open time, setting time, among others, should not be overlooked. However, for this invention, properties such as solubility are imperative since it has a direct impact on the adhesive performance and stability.

The polymeric component of the hot melt adhesive should contain at least one water sensitive thermoplastic component, meaning that the polymer should be partially soluble in water. Soluble polymers for the present invention are polyvinylpyrrolidone /vinyl acetate copolymers (PVP/VA) and ethylene and acrylic acid copolymer (EAA).

The polymeric component content comprises up to 80% by weight of the hot melt adhesive composition.

In a preferred embodiment, where the analyte being detected are nitrite ions, the polymeric component content comprises between 50 and 60% by weight of the hot melt adhesive composition, preferably between 10% and 50%, more preferably between 20% and 40%, and even more preferably between 25% and 350.

### Resin component

The solubility or affinity for water, urine of other bodily fluids, is important to assure a good performance of the product of the invention. For this end, polar raw materials are generally indicated.

Resins for the present invention can be selected from, but not exclusively, aliphatic hydrocarbon resins, aromatic hydrocarbon resins, aromatic modified aliphatic hydrocarbon resins, hydrogenated polycyclopentadiene resins, polycyclopentadiene resins, gum rosins, gum rosin esters, wood rosins, wood rosin esters, tall oil rosins, tall oil rosin esters, polyterpenes, aromatic modified polyterpenes, terpene phenolics, aromatic modified hydrogenated polycyclopentadiene resins, hydrogenated aliphatic resin, hydrogenated aliphatic aromatic resins, hydrogenated terpenes and modified terpenes, hydrogenated rosin acids, hydrogenated rosin esters, derivatives thereof, and combinations thereof.

Despite the considerably lower polarity of hydrocarbon resins compared with the alternatives, namely rosin resins and terpene resins, these resins are preferred for the present invention composition due to the considerably higher stability, lower odour and colour. As such, preferred resins for the present invention comprise hydrocarbon resins, more preferably aliphatic C₅ and aromatic C₉ resins, more exclusively aromatic C₉ resins.

The resin component content comprises up to 60% by weight of the hot melt adhesive composition.

In a preferred embodiment, where the analyte being detected are nitrite ions, the resin component content comprises between 5% and 40% by weight of the hot melt adhesive composition, preferably between 10% and 350, more preferably between 15% and 250.

### Plasticiser component

In the present invention, the plasticiser component allows the melting of the polymer and the adjustment of the viscosity and of the softening point of the hot melt adhesive composition to values that make it easily applicable in industrial lines.

By "acceptable viscosity" is meant a viscosity value at 120 °C comprised between 500 and 15.000 mPa·s, preferably between 750 and 10.000 mPa·s and more preferably between 1.000 and 6.000 mPa·s. By "acceptable softening point" is meant a value comprised between 50 and 120 °C, preferably between 55 and 115 °C and more preferably 60 and 110 °C. Assuring that the composition viscosity and softening point are comprised within the mentioned acceptable values, the working temperature in industrial lines is also assured to be acceptable, specifically, between 70 and 120 °C, preferably between 75 and 120 °C and even more preferably between 80 and 110 °C.

The plasticiser component comprises at least one plasticiser that is liquid at room temperature, to plasticize the polymeric component prior to heating and thereby favour the melting of the polymeric component. Plasticisers for the present invention can be selected from, but not exclusively, inorganic acids and organic acids. A liquid plasticiser at room temperature is a substance with low molecular weight and, as a result, can easily migrate from the hot melt adhesive net and lead to a variation in its properties, such as viscosity. Thus, the plasticiser content should be optimized to effectively plasticize the polymer without boosting the possibility of migration.

As mentioned, polar raw materials are preferred since they provide affinity to water. Considering this, plasticisers with polar functional groups, namely, but not exclusively, organic acids, are preferred. Suitable plasticisers for the present invention can be selected from, but not exclusively, saturated, or unsaturated fatty acids, particularly dimer fatty acids, polymerized fatty acids or derivatives thereof.

The plasticiser component content comprises up to 80% by weight of the hot melt adhesive composition.

In a preferred embodiment, where the analyte being detected are nitrite ions, the plasticiser component content comprises from 50 to 60% by weight of the hot melt adhesive composition, preferably between 10% and 50%, more preferably between 25% and 45%, and even more preferably between 30% and 400.

### Surfactant component

The surfactant component aims to increase the affinity of the composition for water, meaning that it will contribute to the composition solubility. The surfactant component is composed by at least one of the following: non-ionic surfactant, cationic surfactant, anionic surfactant, or amphiphilic surfactant. Also, the hydrophilic-lipophilic balance (HLB) of the surfactant component should be comprised between 3 and 20, preferably between 11 and 20, to assure that it is solubilizing.

Non-ionic surfactants are preferred for the present invention and can be selected from, but not exclusively, natural or synthetic ethoxylated alcohols and ethoxylated phenol derivatives.

The surfactant component content comprises up to 30% by weight of the hot melt adhesive composition.

In a preferred embodiment, where the analyte being detected are nitrite ions, the surfactant component content comprises between 0,5% and 20% by weight of the hot melt adhesive composition, preferably between 1% and 15%, more preferably between 2% and 100, even more preferably between 3% and 5%.

### Reagent component

The reagent component is responsible for detecting the presence of analytes. This detection occurs due to the chemical reaction between the reagent component and the analyte being analysed, which triggers a colour change. Suitable reagents for the present invention include, namely, but not exclusively, Griess Llosvay reagents, Fehling reagents, Benedict reagents, Bradford reagents, Bicinchoninic acid test reagents and Lewis reagents. Reagents of any assay known in the state of the art as being applied to the colorimetric quantification of analytes present in urine can also be added to the reagent component.

In a preferred embodiment, the analyte being detected are nitrite ions, wherein the reagent component consists in Griess Llosvay reagents. Griess Llosvay reagents mainly comprise a first reagent and a second reagent. The first reagent can be selected from, but not exclusively, N-(1-naphthyl)ethylenediamine dihydrochloride and alpha-naphthylamine. The second reagent can be selected from, but not exclusively, sulfanilamide in phosphoric acid, sulfanilic acid, nitroaniline and p-aminoacetophen.

The detection of nitrite ions involves two subsequent reactions: when the second reagent is added to a solution with nitrites, it reacts with a nitrite molecule, forming a diazo (R₂C-N=N) complex that is colourless. Such example of a diazo complex is the nitrite-sulfanilic acid complex. Only then, the diazo complex reacts with the first reagent to obtain a precipitate with red or pink colour. The higher the concentration of nitrites, the stronger the colour. This precipitate can also be referred to as azo dye or azo compound, due to the presence of an azo group (R-N=N-R') in the precipitate molecule.

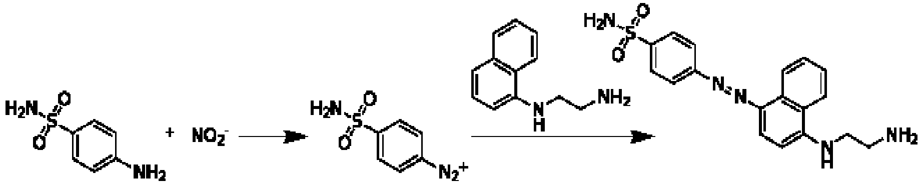

The reagent component content comprises up to 20% by weight of the hot melt adhesive composition.

In a preferred embodiment, where the analyte being detected are nitrite ions, the reagent component content comprises between 1% and 100, preferably between 1% and 80, more preferably between 40 and 60.

### Antioxidant component

The use of an antioxidant component aims to avoid the degradation of the hot melt adhesive due to excessive heat in the course of its production process, as well as during the application and storage of the personal hygiene disposable articles that it is applied on.

A suitable antioxidant component for the present invention comprises at least one primary antioxidant, a secondary antioxidant, or a multifunctional antioxidant. From these, suitable antioxidant components can be selected among phenolic antioxidants, phosphite antioxidants, thioesters-based antioxidants, or mixtures thereof, preferably phenolic.

The antioxidant component content comprises up to 20% by weight of the hot melt adhesive composition.

In a preferred embodiment, where the analyte being detected are nitrite ions, the antioxidant component content comprises between 0,5% and 10% by weight of the hot melt adhesive composition, preferably between 1% and 50, more preferably between 1% and 3%.

### Hot melt adhesive production process

Due to the photosensitive character of the hot melt adhesive composition, the production of this hot melt adhesive should be made in an unlit area, to avoid photodegradation of the composition.

The first step to produce the hot melt adhesive is to blend the reagent component with the plasticiser, while heating slightly (not above 50 °C) to benefit the incorporation of both components, with constant stirring. After the mixture is homogeneous, the polymeric component should be blended in the previous mixture at room temperature until a homogeneous mixture is achieved. Next, the mixture is heated at a maximum temperature of 100 °C with constant stirring until the polymer is completely melted and the mixture turns translucid. The final step is to add the remaining components: resin, surfactant, and antioxidant components, without a specific order. The temperature and stirring should be constant until all the components of the composition are incorporated and the mixture is homogeneous.

### Hot melt adhesive application process

In the production of an article that uses the hot melt adhesive of the present invention, the hot melt should be applied on the backsheet. The article can be, but not exclusively, a personal hygiene disposable article. On the outside of the article a means to protect the hot melt adhesive from light is applied, such as, but not exclusively, an opaque, dark, and easily removable label. Only after usage of the article is the light protection means removed. This ensures that the hot melt adhesive is only exposed to light after the article is used, thereby avoiding premature degradation and ensuring appropriate functionality of the hot melt adhesive.

### Embodiments of the invention

Table 1 presents a suitable embodiment of the present invention. The embodiment of Example 1 relates to a hot melt adhesive composition, where the analyte being detected are nitrite ions, with technical properties such as viscosity, softening point and colour change considered acceptable.

**Table 1: embodiment of Example 1 and its test results.**

| | Example 1 |
|---|---|
| Polymeric component | 32,1 g |
| Resin Component | 19,7 g |
| Plasticiser component | 37,8 g |
| Surfactant component | 3,5 g |
| Antioxidant component | 1,9 g |
| Reagent component | 5,0 g |
| Viscosity at 120 °C | 3.300 mPa·s |
| Softening Point | 65 °C |
| Colour transition | < 3 minutes |

## Claims

1. Hot melt adhesive composition for detecting analytes in urine comprising a polymeric component, a plasticiser component, a resin component, a surfactant, an antioxidant component and an analyte-detecting reagent component **characterized in that** the hot melt adhesive is partially soluble in water and the polymeric component is selected from polyvinylpyrrolidone /vinyl acetate copolymers (PVP/VA) and ethylene and acrylic acid copolymer (EAA).

2. Hot melt adhesive according to the previous claims, wherein the analyte are nitrite ions.

3. Hot melt adhesive according to any of the previous claims, **characterized by** comprising up to 80% by weight of the polymeric component, up to 80% by weight of the plasticiser component, up to 60% by weight of the resin component, up to 30% by weight of the surfactant, up to 20% by weight of the antioxidant component and up to 20% by weight of the analyte-detecting reagent component.

4. Hot melt adhesive according to any of the previous claims, wherein the analyte-detecting reagent component is a Griess-Llosvay reagent.

5. Hot melt adhesive according to any of the previous claims, wherein the resin component comprises at least one hydrocarbon resin.

6. Hot melt adhesive according to any of the previous claims, wherein the plasticiser component comprises at least one liquid plasticiser at room temperature.

7. Hot melt adhesive according to any of the previous claims, wherein the surfactant component comprises at least one surfactant selected from non-ionic surfactants, cationic surfactants, anionic surfactants, or amphiphilic surfactants.

8. Hot melt adhesive according to any of the previous claims, wherein the antioxidant component comprises at least one antioxidant selected from primary antioxidants, secondary antioxidants, or multifunctional antioxidants.

9. Production process of the hot melt adhesive claimed in any of the previous claims, **characterized by** comprising the following steps:
a. blending the plasticiser and the analyte-detecting reagent components, heated to any temperature up to 50 °C;
b. adding the polymeric component at room temperature to the mixture obtained in the previous step, until a homogeneous mixture is obtained;
c. heating the prepared mixture up to 100 °C, with constant stirring, until the polymeric component melts in the mixture and the mixture becomes translucid;
d. adding the resin, surfactant and antioxidant components, without a specific order;
e. maintaining the heating and stirring of step c. until complete incorporation of all the components is observed.

10. Production process according to the previous claim, **characterized by** presenting a final step of application of the hot melt adhesive in an article.

11. Production process according to any of the claims 9 or 10, wherein the process occurs in the absence of light.

12. Production process according to claim 11, wherein a means of light protection is applied to the article.

13. Production process according to claim 12, wherein the light protection means is removed from the article which is a personal hygiene disposable article after use of said article.

14. Use of the hot melt adhesive claimed in any of the claims 1 to 8 in personal hygiene disposable articles.

## Patentansprüche

1. Schmelzklebstoffzusammensetzung zur Detektion von Analyten im Urin, umfassend eine polymere Komponente, eine Weichmacherkomponente, eine Harzkomponente, ein Tensid, eine Antioxidanskomponente und eine analytennachweisende Reagenzkomponente, **dadurch gekennzeichnet, dass** der Schmelzklebstoff teilweise in Wasser löslich ist und die polymere Komponente aus Polyvinylpyrrolidon/Vinylacetat-Copolymeren (PVP/VA) und Ethylen-Acrylsäure-Copolymeren (EAA) ausgewählt ist.

2. Schmelzklebstoff nach den vorhergehenden Ansprüchen, wobei der Analyt Nitrit-Ionen umfasst.

3. Schmelzklebstoff nach einem der vorhergehenden Ansprüche, **dadurch gekennzeichnet, dass** er bis zu 80 Gew.-% einer polymeren Komponente, bis zu 80 Gew.-% einer Weichmacherkomponente, bis zu 60 Gew.-% einer Harzkomponente, bis zu 30 Gew.-% eines Tensids, bis zu 20 Gew.-% einer Antioxidanskomponente und bis zu 20 Gew.-% einer analytnachweisenden Reagenzkomponente umfasst.

4. Schmelzklebstoff nach einem der vorhergehenden Ansprüche, wobei die Analytnachweis-Reagenzkomponente ein Griess-Losvay-Reagenz ist.

5. Schmelzklebstoff nach einem der vorhergehenden Ansprüche, wobei die Harzkomponente mindestens ein Kohlenwasserstoffharz umfasst.

6. Schmelzklebstoff nach einem der vorhergehenden Ansprüche, wobei die Weichmacherkomponente mindestens einen bei Raumtemperatur flüssigen Weichmacher umfasst.

7. Schmelzklebstoff nach einem der vorhergehenden Ansprüche, wobei die Tensidkomponente mindestens ein Tensid umfasst, das aus nichtionischen, kationischen, anionischen oder amphiphilen Tensiden ausgewählt ist.

8. Schmelzklebstoff nach einem der vorhergehenden Ansprüche, wobei die Antioxidanskomponente mindestens ein Antioxidans umfasst, das aus primären, sekundären oder multifunktionalen Antioxidantien ausgewählt ist.

9. Verfahren zur Herstellung des Schmelzklebstoffs nach einem der vorhergehenden Ansprüche, **dadurch gekennzeichnet, dass** es die folgenden Schritte umfasst:
a. Mischen des Weichmachers und der Komponenten des Reagenz zum Nachweis des Analyten, erhitzt auf eine beliebige Temperatur von bis zu 50 °C;
b. Zugabe der polymeren Komponente bei Raumtemperatur zur im vorhergehenden Schritt erhaltenen Mischung, bis eine homogene Mischung erreicht ist;
c. Erhitzen der vorbereiteten Mischung auf bis zu 100 °C bei kontinuierlichem Rühren, bis die polymeren Komponenten in der Mischung schmelzen und die Mischung transluzent wird;
d. Zugabe der Harz-, Tensid- und Antioxidanskomponenten in beliebiger Reihenfolge;
e. Aufrechterhaltung der Temperatur und des Rührens aus Schritt c, bis die vollständige Einarbeitung aller Komponenten zu beobachten ist.

10. Herstellungsverfahren gemäß dem vorhergehenden Anspruch, **dadurch gekennzeichnet, dass** es einen abschließenden Schritt zur Anwendung des Schmelzklebstoffs in einem Artikel umfasst.

11. Herstellungsverfahren gemäß einem der Ansprüche 9 oder 10, wobei der Prozess in Abwesenheit von Licht erfolgt.

12. Herstellungsverfahren gemäß Anspruch 11, wobei ein Lichtschutz auf den Artikel aufgebracht wird.

13. Herstellungsverfahren gemäß Anspruch 12, wobei der Lichtschutz vom Artikel nach der Benutzung entfernt wird und der Artikel der persölichen Hygiene dient.

14. Verwendung des in einem der Ansprüche 1 bis 8 beanspruchten Schmelzklebstoffs in Einwegartikeln für die persönliche Hygiene.

## Revendications

1. Composition adhésive thermofusible pour la détection d'analytes dans l'urine comprenant un composant polymère, un composant plastifiant, un composant résine, un tensioactif, un composant antioxydant et un composant réactif de détection d'analyte **caractérisée en ce que** l'adhésif thermofusible est partiellement soluble dans l'eau et que le composant polymère est sélectionné parmi les copolymères polyvinylpyrrolidone-acétate de vinyle (PVP/VA) et le copolymère éthylène et acide acrylique (EAA).

2. Adhésif thermofusible selon les revendications précédentes dans lequel les analytes sont des ions nitrite.

3. Adhésif thermofusible selon l'une des revendications précédentes, **caractérisé en ce qu'**il comprend jusqu'à 80 % en poids du composant polymère, jusqu'à 80% en poids du composant plastifiant, jusqu'à 60% en poids du composant résine, jusqu'à 30 % en poids du tensioactif, jusqu'à 20% en poids du composant antioxydant et jusqu'à 20% en poids du composant réactif de détection d'analyte.

4. Adhésif thermofusible selon l'une des revendications précédentes, dans lequel le composant réactif de détection d'analyte est un réactif de Griess-Llosvay.

5. Adhésif thermofusible selon l'une des revendications précédentes, dans lequel le composant résine comprend au moins une résine hydrocarbonée.

6. Adhésif thermofusible selon l'une des revendications précédentes, dans lequel le composant plastifiant comprend au moins un plastifiant liquide à température ambiante.

7. Adhésif thermofusible selon l'une des revendications précédentes, dans lequel le composant tensioactif comprend au moins un tensioactif sélectionné parmi les tensioactifs non ioniques, les tensioactifs cationiques, les tensioactifs anioniques ou les tensioactifs amphiphiles.

8. Adhésif thermofusible selon l'une des revendications précédentes, dans lequel le composant antioxydant comprend au moins un antioxydant sélectionné parmi les antioxydants primaires, les antioxydants secondaires ou les antioxydants multifonctionnels.

9. Procédé de production de l'adhésif thermofusible revendiqué dans l'une des revendications précédentes, **caractérisé en ce qu'**il comprend les étapes suivantes:
a. mélange du plastifiant et des composants réactifs de détection d'analyte, chauffé à une température allant jusqu'à 50 °C;
b. ajout du composant polymère à température ambiante au mélange obtenu à l'étape précédente jusqu'à l'obtention d'un mélange homogène;
c. chauffage du mélange préparé à une température allant jusqu'à 100 °C, en remuant constamment, jusqu'à ce que le composant polymère fonde dans le mélange et que le mélange devienne translucide;
d. ajout de la résine, du tensioactif et des composants antioxydants, sans respecter un ordre spécifique;
e. continuer à chauffer et à remuer comme indiqué à l'étape c. jusqu'à ce qu'on observe que tous les composants sont complètement incorporés.

10. Procédé de production selon la revendication précédente, **caractérisé en ce qu'**il présente une dernière étape d'application de l'adhésif thermofusible sur un article.

11. Procédé de production selon l'une des revendications 9 ou 10, dans lequel le procédé se produit en l'absence de lumière.

12. Procédé de production selon la revendication 11, dans lequel un moyen de protection légère est appliqué sur l'article.

13. Procédé de production selon la revendication 12, dans lequel le moyen de protection légère est retiré de l'article, lequel est un article jetable d'hygiène personnelle, après utilisation dudit article.

14. Utilisation de l'adhésif thermofusible, revendiquée dans l'une des revendications 1 à 8, dans les articles jetables d'hygiène personnelle.
